# EUROPÄISCHE PATENTSCHRIFT

(11) **EP 1 275 405 B1**
(45) Veröffentlichungstag und Bekanntmachung des Hinweises auf die Patenterteilung: **11.10.2006**
(21) Anmeldenummer: 02011899.8
(22) Anmeldetag: 29.05.2002
(51) Int. Cl.: A61L 27/28, A61L 27/56

(54) **Implantat mit poröser Proteinmatrix und Verfahren zu seiner Herstellung**
Implant with porous protein matrix and process for its production
Implant avec des matrices protéiques poreuses et leur procédé de fabrication

(30) Priorität: 13.07.2001 DE 10135275
(43) Veröffentlichungstag der Anmeldung: 15.01.2003
(73) Patentinhaber: JOTEC GmbH, 72379 Hechingen (DE)
(72) Erfinder: Simmoteit, Robert, Dr., 72414 Rangendingen (DE); Fischer, Heike, Dr., 72762 Reutlingen (DE)
(74) Vertreter: Otten, Hajo

(56) Entgegenhaltungen:
- EP-A- 0 562 864
- WO-A-00/01432
- WO-A-00/47129
- WO-A-92/10217
- WO-A-99/27315
- WO-A1-00/38590
- DE-A- 3 203 957
- JP-A- 8 024 326
- US-A- 4 787 900
- US-A- 5 716 411

## Beschreibung

Die Erfindung betrifft ein Implantat sowie ein Verfahren zu seiner Herstellung.

Implantate und Verfahren zu ihrer Herstellung sind aus dem Stand der Technik vielfach bekannt.

Implantierbare Strukturen in Kombination mit Zellen finden ihre Anwendung vor allem im Gebiet des Tissue Engineering, einem interdisziplinären Forschungsgebiet, das sich mit Methoden und Materialien zur Herstellung von "künstlichen" Gewebe- und Organsystemen befaßt. So können künstlich hergestellte Implantate z.B. als Haut-, Knochen-, Knorpel-, Linsen- oder Gefäßersatz Anwendung finden.

In der Gefäßchirurgie werden kleinlumige Implantate vor allem dann eingesetzt, wenn die körpereigenen Gefäße eines Patienten nicht verwendbar sind. Dies ist z.B. der Fall, wenn eine spezifische Gefäßlänge benötigt wird, oder wenn die autologen Gefäße aufgrund pathophysiologischer Eigenschaften nicht einsetzbar sind. Hier kommen Gefäßprothesen aus Kunststoff zum Einsatz, wobei vor allem Kunststoffe, wie z.B. gewirkte Fäden aus Polyethylenterephthalat (PET), (Handelsname: Dacron), oder aus expandiertem Polytetrafluorethylen (ePTFE) verwendet werden.

Gefäßimplantate aus diesen Kunststoffen werden bevorzugt verwendet, da sie vorteilhafte strukturelle und biokompatible Eigenschaften besitzen. So kann einerseits umliegendes Gewebe einwachsen, andererseits darf durch die Poren kein Blutplasma austreten. Dies wird bei den ePTFE-Implantaten durch die eingestellte Größe der Poren erzielt, während gewirkte PET-Implantate durch eine Beschichtung mit resorbierbaren Materialen wie z.B. Kollagen oder Gelatine imprägniert werden. Nach Implantation wird die Beschichtung in dem Maße resorbiert, indem das umliegende neu gebildete Gewebe in die poröse Kollagenschicht einwächst.

Werden einseitig außen kollagenbeschichtete Kunststoffe verwendet, besteht z.B. bei kleinlumigen Gefäßprothesen die große Gefahr, daß durch die freiliegende Fremdoberfläche des Innenlumens die Blutgerinnung induziert und das implantierte Gefäß sehr schnell verschlossen wird. Denn insbesondere der Kontakt von langsam strömenden Blut mit künstlichen Oberflächen kann zur Aktivierung des Gerinnungssystems, des Komplementsystems und des Immunsystems führen.

Somit können solche Prothesen nicht im Bereich kleinlumiger Gefäße (∅ < 6 mm) verwendet werden, da hier die Gefahr eines schnellen Gefäßverschlusses besteht.

Weitergehende Ansätze zur Vermeidung von Blutgerinnung gehen dahin, die beschichteten Implantate mit Zellen, wie z.B. Endothelzellen oder Fibroblasten, zu besiedeln.

Endothelzellen kleiden die Oberfläche der menschlichen Blutgefäße aus. Die Besiedelung des Lumens von Gefäßprothesen mit Endothelzellen bietet dem strömenden Blut eine Oberfläche mit deutlich reduzierten gerinnungs- und komplement-aktivierenden Eigenschaften. Die Kokultivierung von Gefäßprothesen mit Fibroblasten und Endothelzellen - wie z.B. in der PCT 98/01782 gezeigt - ermöglicht ein verbessertes Einwachsen des Implantats in das umliegende Gewebe sowie eine Stabilisierung der Endothelzellschicht im Innenlumen.

Somit ist die Interaktion der verschiedenen Zellarten, die beispielsweise in natürlichen Gefäßen vorhanden sind, für die Funktionalität der Implantate bedeutend. Neben einer hohen Biokompatibilität muß auch gewährleistet sein, daß die Struktur des Implantats an die Anforderungen von verschiedenen Zellen angepaßt ist.

Ein weiterer Entwicklungsansatz ist die Verwendung von azellularisierten Gefäßen tierischen Ursprungs. In der US 5 899 936 werden diese Gefäße nach dem Entfernen ihrer zellularen Bestandteile mit autologen Zellen besiedelt und anschließend implantiert.

Einen besonderen Nachteil dieser Entwicklungen stellt die Tatsache dar, daß die Implantate nicht dem Patienten angepaßt hergestellt werden können, sondern in bezug auf Länge und Größe des Gefäßes durch das Spendertier vorgegeben sind. Des weiteren sind die Risiken einer Krankheitsübertragung durch Viren oder Prionen über solche Gewebe nicht vollständig geklärt.

Ein weiterer Nachteil besteht darin, daß es nicht möglich ist, in ein derart hergestelltes Implantat zur Erhöhung der Stabilität eine zusätzliche Stützstruktur einzubetten. Außerdem sind azellularisierte Strukturen nur bedingt lagerbar. Die neue Diskussion im Bereich der Xenotransplantate führt ferner dazu, daß technisch hergestellte Gefäßprothesen bevorzugt werden.

Neuere Versuchsansätze gehen dahin, vollständig resorbierbare Implantate zu entwickeln, die aus Kunststoffen wie Polyglykolsäure oder Polylactid bestehen. Diese Kunststoffe werden im Rahmen der Wundheilung durch körpereigenes Gewebe ersetzt. Ein Vorteil dieser Implantate besteht darin, daß die Materialien vollständig regenerieren und keine Kunststoffe im Körper verbleiben, die Infektionen hervorrufen können. Nachteile dieser Implantate sind die Beeinträchtigung des Zellwachstums während der Resorption z.B. durch eine pH-Wert-Verschiebung beim Abbau von Polylactid sowie die schwierige Steuerung der Gewebeneubildung, da eine frühzeitige Resorption zu einem Implantatversagen führen kann.

Aus der WO 00/47129 ist ein Verfahren bekannt, mittels einer Schablone eine resorbierbare Membran herzustellen, die eine dreidimensionale Struktur besitzt. Die Membran kann dabei auch aus nicht-resorbierbaren Kunststoffen gefertigt werden und gegebenenfalls mit einer Proteinmatrix beschichtet sein.

Dieses Verfahren hat den Nachteil, daß die Herstellung der dreidimensional-strukturierten Membran sehr aufwendig ist. So müssen zuerst die Schablonen für die jeweils benötigten unterschiedlichen Membranen gefertigt werden, bevor die Stützstruktur selber produziert werden kann.

Die US 4 787 900 offenbart ein Verfahren, bei dem eine innere Struktur aus einem resorbierbaren Material mit einer äußeren Schicht aus einem ebenfalls abbaubaren Material beschichtet wird. Beide Schichten können dabei jeweils mit Zellen besiedelt sein. Nachteilig bei diesem Verfahren ist die Tatsache, daß die äußere Struktur den jeweiligen Anforderungen der Umgebung, in die sie implantiert werden soll, nicht angepaßt werden kann. Außerdem ist die Herstellung der äußeren Schicht bei diesem Verfahren mit aufwendigen Schritten verbunden, da die äußere Struktur nach einem Gefriertrocknungsprozeß erst auf die erwünschte Schichtdicke zurückgeschnitten werden muß, was zu einem beträchtlichen Materialverbrauch führt.

An Implantate werden also besondere mechanische und strukturelle Anforderungen gestellt. So sollen sie neben einer ausreichenden Strukturstabilität auch ein auf das zu ersetzende Gewebe abgestimmtes Kraft- und Dehnungsverhalten besitzen. Implantate müssen außerdem verschiedene Paßformen, Längen und Durchmesser besitzen. Zudem spielt die Mikrostrukturierung, wie z.B. die innere Porenstruktur, eine wichtige Rolle für die Besiedelung mit Zellen und für einwachsendes Gewebe. Die Implantate sollen sich des weiteren dadurch auszeichnen, daß sie keine immunologischen Allergien oder Reaktionen auslösen und daß sie dem jeweiligen Gewebe, in das sie implantiert werden, optimal angepaßt sind.

Der Erfindung liegt daher die Aufgabe zugrunde, ein Implantat sowie ein Verfahren zu seiner Herstellung bereitzustellen, wobei das Implantat mit verschiedenen Zellarten besiedelt und eine Abkapselung der Fremdstruktur oder - im Falle einer Gefäßprothese - ein Verstopfen verhindert wird.

Bei dem eingangs genannten verfahren wird diese Aufgabe erfindungsgemäß gelöst durch die Herstellung eines Implantats mit einer strukturierten Fremdstruktur und einer in der strukturierten Fremdstruktur zumindest teilweise verankerten porösen Proteinmatrix mit gerichteter Porenstruktur, wobei als Fremdstruktur ein Material verwendet wird, das ausgewählt ist aus einem strukturierten beständigen künstlichen Material, einem strukturierten resorbierbaren künstlichen Material und einem strukturierten natürlichen Material oder Mischungen der genannten Materialien; das Verfahren weist dabei die folgenden Schritte auf: a) Bereitstellen der Fremdstruktur, b) Auftragen einer Suspension, Dispersion oder Paste mit Kollagen und löslichen, nicht-kollagenen Bestandteilen, c) zumindest teilweises Einbringen der Suspension, Dispersion oder Paste in die Fremdstruktur durch Druck, Vakuum oder Zentrifugation, d) einseitiges Abkühlen einer Oberfläche der Suspension, Dispersion oder Paste und gleichzeitiges Isolieren der anderen Oberfläche.

Ferner wird die Aufgabe gelöst durch ein Implantat mit in einer strukturierten Fremdstruktur zumindest teilweise verankerten Proteinmatrix mit gerichteter Porenstruktur gelöst, wobei als Fremdstruktur ein Material verwendet wird, das ausgewählt ist aus einem strukturierten beständigen künstlichen Material, einem strukturierten resorbierbaren künstlichen Material und einem strukturierten natürlichen Material oder Mischungen der genannten Materialien.

Die Fremdstruktur kann verschiedene Funktionen übernehmen, wie z.B. eine Stützfunktion oder die Funktion einer Barriere gegen Feuchtigkeitsverlust und Infektion. Letzteres kann bspw. bei einem Hautimplantat erforderlich sein. Eine weitere mögliche Funktion der Fremdstruktur ist die Nährstoffversorgung in großlumigen Implantaten. Hierzu kann die Fremdstruktur als Hohlfasernetz ausgebildet sein, zumindest aber einige Hohlfasern enthalten.

Bei einer Besiedelung des erfindungsgemäßen Implantats mit Zellen können diese zügig entlang den Proteinfasern in die Matrix einwandern, da die Porenstruktur und Porengröße spezifisch für die jeweilige Zellart eingestellt werden kann.

Durch die Verankerung der Proteinmatrix mit gerichteter Porenstruktur in der Fremdstruktur wird außerdem eine ausreichende Stabilität des gesamten Implantats gewährleistet.

Bei einer bevorzugten Ausgestaltung des erfindungsgemäßen Verfahrens wird als strukturierte Fremdstruktur ein beständiges künstliches Material verwendet, das aus einer Gruppe, umfassend Polytetrafluorethylen, Polyurethan, Polystyrol, Polyester, Keramik oder Metalle ausgewählt ist.

Expandiertes Polytetrafluorethylen (ePTFE) hat sich in den letzten Jahren als bevorzugtes künstliches Material für Implantate durchgesetzt. Dieses Material ist porös und derart gestaltet, daß bei einer Implantation die Zellen nicht einwachsen können. Bei diesen, dem Stand der Technik entsprechenden Gefäßeprothesen ist durch die Beschaffenheit der Poren auch dafür gesorgt, daß keine (Blut-) Flüssigkeit durch die Poren austreten kann.

In einer anderen bevorzugten Ausgestaltung des erfindungsgemäßen Verfahrens wird als strukturierte Fremdstruktur ein resorbierbares künstliches Material verwendet, das aus der Gruppe, umfassend Polylactid, Poly-Glykolsäure, Polyhydroxyalkanoate oder deren Copolymere ausgewählt ist.

Dabei ist nicht ausgeschlossen, daß als strukturiertes Fremdmaterial auch andere reversible natürliche Materialien, ausgewählt aus einer Gruppe, umfassend Chitin, Cellulose, Kollagen oder Hydroxylapatite (Calciumphosphat), verwendet werden. Diese können z.B. in Form einer oberflächlich strukturierten Folie als Fremdmaterial eingesetzt werden. Zu den verwendbaren Calcium-phosphat-Verbindungen zählen bspw. Apatit, Tricalciumphophat und Tetracalciumtriphosphat, kombiniert mit Calciumhydroxid.

Alternativ kann die strukturierte Fremdstruktur aus Materialkombinationen der oben genannten Materialien zusammengesetzt sein.

Die Form der Fremdstruktur kann beliebig gewählt sein. Sie kann somit z.B. als Hautimplantat eben ausgewählt sein, oder als Gefäßimplantat tubulär ausgestaltet sein, wobei diese tubuläre Ausgestaltung beliebig geformt sein kann, z.B. als verzweigter oder unverzweigter Tubus etc. Für andere Anwendungszwecke wie z.B. Knorpel, Knochen kann sie auch als Zylinder, Rechteck oder Herzklappe gestaltet sein. Wenn die Fremdstruktur Hohlfasern umfaßt, könnten diese in die Proteinmatrix eingebettet sein und als Leitungen für die Nährstoffversorgung von Zellen dienen, mit denen die Proteinmatrix besiedelt werden kann.

Bei dem erfindungsgemäßen Verfahren wird die Proteinmatrix aus einer Suspension, Dispersion oder Paste mit Kollagen und löslichen, nicht-kollagenen Bestandteilen hergestellt.

Kollagen ist das häufigste Protein im menschlichen Körper und stellt einen wesentlichen Bestandteil der extrazellulären Matrix von Haut, Gefäßen, Knochen, Sehnen, Knorpel, Zähnen etc. dar. Die Verwendung von nativem Kollagen ist vorteilhaft, da Kollagen als Biomaterial eine Vielzahl an positiven Eigenschaften aufweist.

Nicht-kollagene Begleitstoffe können Wachstumsfaktoren, Wirkstoffe oder andere Bestandteile der extrazellulären Matrix, wie z.B. Elastin, Laminin, Fibronektin, Hyaluronsäure, Glykosaminoclycane sein. Die löslichen Bestandteile umfassen einerseits Säuren wie HCl, Essigsäure oder Ascorbinsäure, da bekannt ist, daß der optimale pH-Bereich zur Herstellung gefriergetrockneter Kollagenschwämme zwischen 2,5 und 3,5 liegt. Andererseits können lösliche Zusatzstoffe wie Glycerin oder Ethanol oder feindispergierte wie z.B. Calciumphosphat etc. als Begleitstoffe eingesetzt werden, da durch ihre Konzentration die Eiskristallmorphologie und somit die Porenstruktur eingestellt werden kann.

Bei dem erfindungsgemäßen Verfahren wird die Suspension gleichmäßig auf die Fremdstruktur aufgebracht und durch Druck, Vakuum oder Zentrifugation zumindest teilweise in diese eingebracht.

Durch das gleichmäßige Aufbringen der Suspension wird eine einheitliche Schichtdicke gewährleistet, was ein nachträgliches Bearbeiten oder Zuschneiden unnötig macht und somit zusätzliche Arbeitsschritte vorteilhaft erspart. Dabei können Wandstärken zwischen 0,1 cm und 5 cm eingestellt werden. Druck, Vakuum oder Zentrifugation können derart geregelt werden, daß die Suspension kontrolliert und mit einer bestimmten Tiefe in die strukturierte/poröse Fremdstruktur eingebracht wird. Dabei ist es von Vorteil, wenn die Probe während dieses Prozesses temperiert wird, da die Viskosität der Suspension temperaturabhängig ist.

Ferner wird bei dem erfindungsgemäßen Verfahren die gerichtete Porenstruktur der Proteinmatrix durch einseitiges Abkühlen einer Oberfläche und gleichzeitiges Isolieren der anderen Oberfläche ausgebildet. Dabei wird die Proteinsuspension kontinuierlich oder stufenweise auf einer Seite kontrolliert abgekühlt, wobei die andere Seite durch einen Isolator, beispielsweise Luft, Gase oder Teflon abgegrenzt wird. Während des Einfrierprozesses kann die Suspension teilweise oder vollständig auskristallisieren. Die Kollagene und gelösten Stoffe werden dabei größtenteils von der wachsenden zellularen Eisphasenfront verschoben, wobei die suspendierten Proteine und die gelösten oder dispergierten Stoffe zwischen den Eiskristallen hoch aufkonzentriert werden. Durch die Kühlrate und die chemische Zusammensetzung der Proteinsuspension können die Eiskristallstruktur und -größe bei einer beliebigen Schichtdicke vorteilhaft eingestellt werden.

Die WO 99/27315 offenbart ein Einfrierverfahren, wobei aber jeweils an zwei gegenüberliegeiden Seiten gleichmäßig heruntergekühlt wird. Diese Druckschrift führt jedoch aus, daß ein einseitiges Abkühlen nachteilig ist und zu keiner gerichteten Porenstruktur führt.

Wie jedoch in dem erfindungsgemäßen Verfahren gezeigt werden konnte, wachsen auch während des einseitigen Einfrierprozesses die Eiskristalle nahezu parallel zu dem Temperaturgradienten gerichtet durch die Probe. Dadurch, daß die Proteinsuspension in die Fremdstruktur eingebracht ist, wachsen die Eiskristalle auch teilweise in die Fremdstruktur ein.

Die gerichtete Porenstruktur ist erwünscht, da die Struktur somit dem jeweiligen Gewebe optimal angepaßt werden kann. Eine Anpassung der Porenstruktur und -größe verbessert das Migrationsverhalten des umliegenden Gewebes.

Dabei ist bevorzugt, wenn die Porengröße der Proteinmatrix gezielt zwischen 5 µm und 500 µm eingestellt wird.

In einer Weiterführung des erfindungsgemäßen Verfahrens ist bevorzugt, wenn die Probe nach dem Abkühlen gefriergetrocknet wird.

Bei dem Gefriertrocknungsprozeß sublimieren die Eiskristalle, wodurch Hohlräume entstehen, die den gerichteten Poren der Struktur entsprechen. Bei einer Implantation können die Zellen des umliegenden Gewebes also entlang den Proteinfasern in das Implantat einwachsen. Durch den Wasserentzug während der Sublimation bilden sich kovalente Bindungen zwischen den Kollagenmolekülen aus, wodurch die Matrix eine erwünschte Stabilität erhält. Dabei ist es vorteilhaft, daß der Vernetzungsgrad durch den Gefriertrocknungsprozeß oder durch eine chemische Behandlung des gefriergetrockneten Produktes gezielt eingestellt werden kann. Die durch den Gefriertrocknungsprozeß entstandene Proteinmatrix ist direkt mit der Fremdstruktur verankert, und es sind keine weiteren Schritte zur Verbindung der Fremdstruktur mit der Proteinmatrix nötig.

Bevorzugt ist weiterhin, wenn die Struktur nach der Gefriertrocknung sterilisiert wird. Dies ist eine erwünschte Voraussetzung zur Lagerung oder direkten Verwendung der Strukturen.

Die Struktur kann nun direkt implantiert werden, oder aber in einer bevorzugten Ausführung des erfindungsgemäßen Verfahrens mit Zellen besiedelt werden. Als Zellen können hierbei xenogene, allogene oder autologe Spenderzellen, Stammzellen oder Zellinien eingesetzt und anschließend kultiviert werden. Diese Besiedelung wird dabei vorzugsweise in einem Bioreaktor unter physiologischer Belastung durchgeführt, wobei je nach Anwendung eine Co-Kultivierung der im natürlichen Gewebe enthaltenen Zellarten vorgesehen ist, die zudem zu unterschiedlichen Zeitpunkten auf die Struktur aufgebracht werden können.

Die Struktur kann dabei bis zur festen Adhäsion der Zellen kultiviert oder aber so lange inkubiert werden, bis ein Ab- bzw. Umbau der resorbierten Bestandteile stattgefunden hat. Die Kultivierungsdauer ist von der jeweiligen Form der Struktur und von den eingesetzten Zellen abhängig.

Dies hat den Vorteil, daß beispielsweise Gefäßimplantate erst mit einer inneren Epithelzell-Schicht versehen werden können, wodurch vor allem beim Einsatz von autologen Spenderzellen Thrombogenisierung und Kalzifizierung des Implantats vermieden werden.

Die Struktur kann außerdem je nach Bedarf vor der Implantation mit Wirkstoffen beladen bzw. beschichtet werden, wie beispielsweise Hirudin, Aspirin, Heparansulfat, Albumin oder ähnlichem. Die Wirkstoffabgabe kann vorzugsweise durch eine zusätzlich aufgebrachte Hydrogelbeschichtung oder durch die Art der Anbindung der Wirkstoffe kontrolliert werden.

Durch die Erfindung wird mit einfachen Mitteln erreicht, daß das Implantat den jeweiligen Anforderungen des umliegenden Gewebes durch die Ausbildung einer gerichteten Porenstruktur einfach und effizient angepaßt werden kann. Gleichzeitig zeichnet sich ein nach dem erfindungsgemäßen Verfahren hergestelltes Implantat durch mechanische Stabilität und biologische Kompatibilität aus. Zudem kann sie in sehr unterschiedlichen geometrischen Formen hergestellt werden und so neben anderen Einsatzbereichen auch bei pharmazeutischen und kosmetischen Anwendungen sowie in der Geweberekonstruktion eingesetzt werden.

Das Verfahren bietet die Möglichkeit, in wenigen Schritten und ohne technischen oder materiellen Aufwand gut resorbierbare Implantate mit einem geringen Fremdmaterialanteil herzustellen.

Die einzusetzenden Mengen an Material, vor allem die der Proteinmatrix, können genau berechnet werden, so daß ein unnötiger Materialverbrauch vermieden wird. Dadurch kann außerdem gleichzeitig die erwünschte Schichtdicke des Implantats eingestellt werden. Mit dem Verfahren lassen sich Schichtdicken der Proteinmatrix zwischen 0,1 und 5 cm herstellen.

Die Ausbildung einer gewünschten Porenstruktur kann leicht berechnet und mit einfachen Mitteln ausgeführt werden, so daß eine aufwendige Konstruktion von z.B. Schablonen entfällt, was eine deutliche Zeit- und Kostenersparnis zum Vorteil hat.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine detaillierte Schnittdarstellung eines Ausführungsbeispiels eines erfindungsgemäßen Implantats;
- Fig. 2: einen Querschnitt eines weiteren Ausführungsbeispiels eines erfindungsgemäßen Implantats, nämlich einer Gefäßprothese;
- Fig. 3: ein Beispiel für den Temperaturverlauf bei einseitig geregelter Temperierung;
- Fig. 4: eine Vorrichtung zur Herstellung eines Implantats mit gerichteter Porenstruktur.
- Fig. 5: einen vergrößerte Schnittdarstellung der Vorrichtung aus Fig. 4

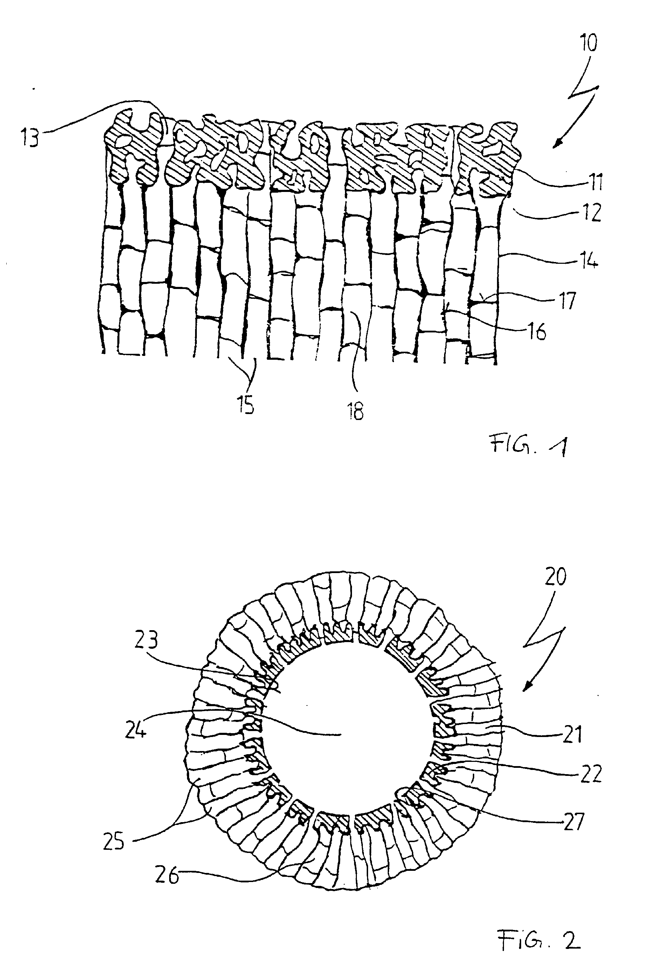

In Fig. 1 bezeichnet 10 insgesamt ein Implantat mit einer inneren Fremdstruktur 11 mit geschlossenen Poren 12 und durchgängigen Poren 13 und außerdem mit einer äußeren Proteinmatrix 14, die aus langen Proteinfasern 15 besteht. Die Proteinfasern 15 liegen in dünnen Proteinfolien 16 und einzelnen kurzen Proteinfasern 17 vor, die zusammen Poren 18 bilden.

Während des Herstellungsprozesses verankert sich die Proteinmatrix 14 in der inneren Fremdstruktur 11, die durch die geschlossenen und durchgängigen Poren 12 und 13 strukturiert ist. Die Proteinmatrix 14 besteht aus kovalent vernetzten Proteinfasern 15, die aufgrund des noch zu beschreibenden Herstellungsverfahrens und der Anisotropie von Eis sehr dünne Proteinfolien 16 bilden und durch einzelne kurze Proteinfasern 17 miteinander verbunden sind. Die durchgängigen Poren 13 der Fremdstruktur 11 sind groß verglichen zu den Poren 18 der Proteinmatrix 14, so daß sich die Struktur der Proteinmatrix 14 in den Poren 13 der Fremdstruktur 11 ungehindert fortsetzen kann. Die Proteinmatrix 14 wird durch die direkte Verbindung mit der Fremdstruktur 11 gegenüber mechanischen Belastungen stabilisiert. In den Poren 18 der Proteinmatrix 14 können Zellen und Flüssigkeiten aufgenommen werden, wobei die Proteinfolie 16 und die kurzen Proteinfasern 17 hierbei Adhäsionsflächen für die Zellen bilden. Bei einer Verwendung von Kollagen als Proteinbestandteil können die angehefteten Zellen das resorbierbare Trägermaterial in körpereigene extrazellulare Matrix umbauen.

In Fig. 2 ist eine Gefäßprothese 20 gezeigt, die nach dem erfindungsgemäßen Verfahren hergestellt wurde. Bei diesem Implantat ist eine Proteinmatrix 21 in einer Fremdstruktur 22 tief verankert. Eine Innenwand 23 der Gefäßprothese 20 bildet die Grenze zum Lumen 24. Die Proteinmatrix 21 besteht aus Proteinfasern 25 mit Poren 26. Die Proteinfasern 25 stehen über durchgängige Poren 27 der Fremdstruktur 22 mit dem Lumen 24 in direktem Kontakt.

Durch die Fremdstruktur 22 wird der Prothese 20 eine ausreichende Stabilität verliehen. In die gerichtet verlaufenden Poren 26 der Proteinmatrix 21 können Zellen wie z.B. Fibroblasten zügig einwachsen. Zusätzlich kann die Innenwand 23 der Gefäßprothese 20 mit Endothelzellen besiedelt werden, um eine Thrombogenität der Prothese zu vermeiden.

Es darf an dieser Stelle angemerkt werden, daß die in Fig. 2 gezeigte Gefäßprothese 20 nur ein Ausführungsbeispiel verkörpert. Die Erfindung ist daneben auch in anderen Formen und Funktionen einsetzbar, wie z.B. als Patches bei Hautimplantaten, als Zylinder oder Rechteck bei Knorpel- und Knochenimplantaten oder Herzklappen.

Durch das Verfahren können Implantate mit unterschiedlichen Schichtdicken der Proteinmatrix hergestellt werden. Um eine bestimmte Proteinmatrix-Schichtdicke zu erreichen, wird ein bestimmter Temperaturverlauf angelegt.

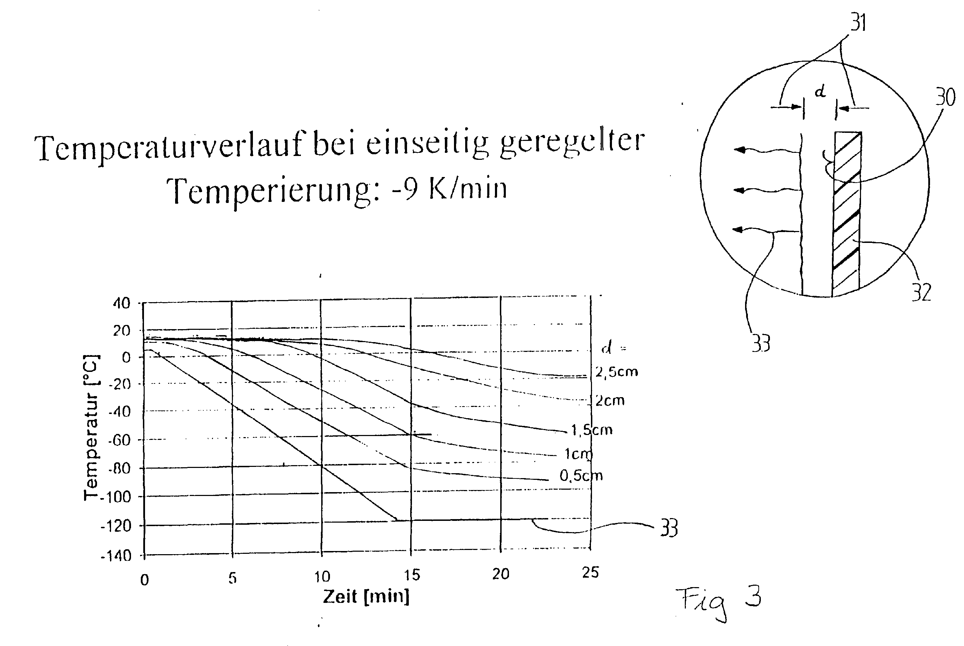

Fig. 3 zeigt den Temperaturverlauf bei einer konstanten Abkühlrate einer Kollagensuspension von -9 K/min in verschiedenen Schichtdicken, aufgetragen über die Zeit. Die Temperaturverläufe wurden in folgenden Schichtdicken d gemessen: 2,5; 2; 1,5; 1 und 0,5 cm. Der Meßpunkt 30 für die Temperaturverläufe ist in dem Ausschnitt rechts neben dem Diagramm schematisch dargestellt. Der von den Pfeilen 31 begrenzte Bereich d gibt die Schichtdicke der Proteinmatrix (hier bestehend aus Kollagen) wieder. Mit 32 ist ein Kunststoff dargestellt, der als Fremdstruktur dient. Die Pfeile 33 geben die Abkühlrichtung an. So sind z.B. für eine Wandstärke von 1 cm zwischen 10 und 15 min nötig, um -50 °C zu erreichen. Die unterste Kurve 33 des Diagramms zeigt den Temperaturverlauf für Wandstärken < 1 mm. Für diese Wandstärken ist durch Extrapolation eine Abkühlzeit von 5-10 min zu erwarten, um -50 °C zu erreichen. Unter diesen Bedingungen wachsen Eiskristalle einer Größe von ca. 35 µm im Durchmesser durch die Kollagensuspension.

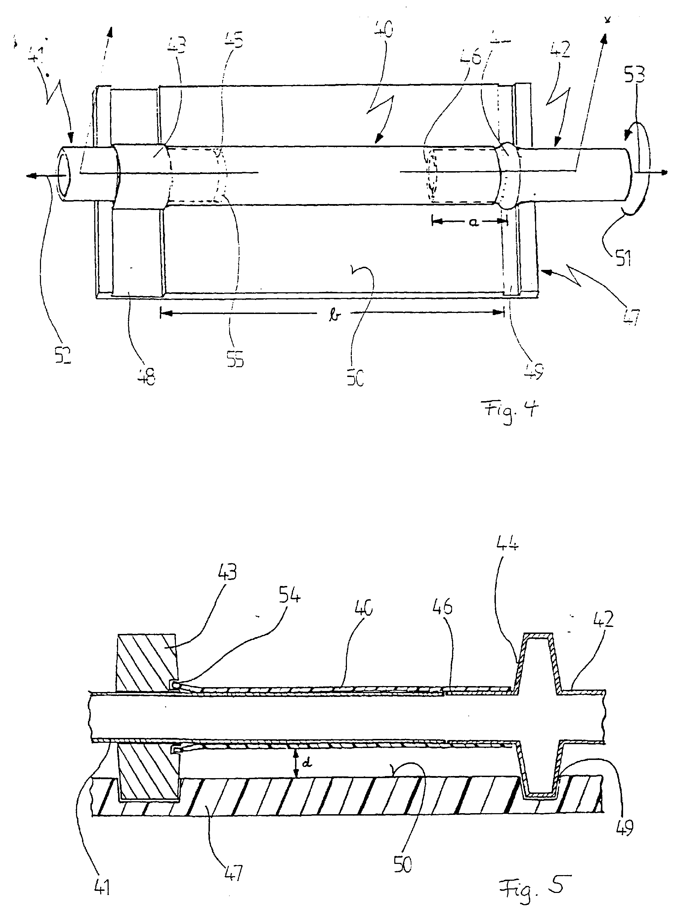

Fig. 4 zeigt eine Vorrichtung zur Herstellung eines Ausführungsbeispiels des erfindungsgemäßen Verfahrens, nämlich einer Gefäßprothese. Eine schlauchförmige, vorgefertigte Fremdstruktur 40 wird an ihren Enden jeweils auf zwei Metallrohre 41 und 42 gespannt, wobei auf dem Metallrohr 41 ein Ring 43 befestigt ist und das Metallrohr 42 einen Flansch 44 aufweist. Der Ring 43 und Flansch 44 sind jeweils in einem bestimmten Abstand a von den Enden der Metallrohre 44 und 45 entfernt.

Die Vorrichtung zeigt außerdem eine Folie 47, die durchgehende Vertiefungen 48 und 49 besitzt, deren Ausmaß dem Ring 43 und dem Flansch 44 entspricht. Der Abstand b der Vertiefungen entspricht der Länge der Anordnung aus dem Ring 43 und dem Flansch 44 und der dazwischengespannten Fremdstruktur 40. Die Folie 47 kann auf ihrer zwischen den Vertiefungen 48 und 49 liegenden Fläche 50 mit einer Proteinsuspension beschichtet werden und anschließend um die Anordnung aus Metallrohren 41, 42 und der dazwischengespannten Fremdstruktur 40 gewickelt werden, was durch einen Pfeil 51 angedeutet ist. Die Metallrohre 41 und 42 können über ihre jeweiligen freien Enden mit einer Vakuumpumpe verbunden werden, wie durch die Pfeile 52 und 53 gezeigt ist.

In Fig. 5 ist eine vergrößerte Schnittdarstellung der Vorrichtung aus Fig. 4 gezeigt. Es wurden gleiche Bezugszeichen wie in Fig. 4 verwendet, sofern sie sich auf dieselben Merkmale beziehen.

In die Vertiefung 49 der Folie 47 ist der Flansch 44 des Metallrohres 42 eingebracht. Über das Ende 46 des Metallrohrs 42 ist die schlauchförmige Fremdstruktur 40 gezogen. In der Abbildung ist das Metallrohr 42 durch den Ring 43 in die schlauchförmige Fremdstruktur 40 bis zum Ende des Metallrohres 46 eingebracht. Zwischen der Oberfläche 50 der Folie 47 und der schlauchförmigen Fremdstruktur 40 ist mit d die Schichtdicke der Proteinsuspension angegeben, die durch den Abstand der Folie 47 von der schlauchförmigen Fremdstruktur 40 bestimmt wird. Diese Schichtdicke kann durch Ändern der Tiefe der Vertiefungen 48 und 49 oder der Länge des Flansches 44 und des Ringes 43 beliebig eingestellt werden.

Anhand des unten beschriebenen Beispiels wird die Anwendung der Vorrichtung gezeigt.

### Beispiel: Herstellung einer erfindungsgemäßen Gefäßprothese

Nach bekannten Verfahren wird eine schlauchförmige Fremdstruktur 40 aus expanded Polytetrafluorethylen (ePTFE) mit einem Innendurchmesser von 4 mm und einer Wandstärke von 100 µm hergestellt. Durch Verstreckung wird eine sehr hohe Porosität erzielt, so daß die mittlere Porengröße, bestimmt durch den Abstand der PTFE-Knoten, 60 µm beträgt.

Die Enden des Schlauches, dessen Gesamtlänge 340 mm beträgt, werden jeweils mit 15 mm auf konzentrisch doppellumige Metallrohre 41, 42 mit Außendurchmesser 4,1 mm geschoben. Das Metallrohr 42 besitzt im Abstand von 15 mm von seinem Ende 46 einen Flansch 44. Auf dem zweiten Rohr befindet sich ein Ring 43, der durch eine Muffe auf das Rohr geklemmt werden kann. Der Ring 43 besitzt eine Klemmvorrichtung 54, in die das eine Ende der Fremdstruktur 40 in den Ring 43 eingeklemmt werden kann. Die freien Enden der Metallrohre können mit einer Vakuumpumpe verbunden werden.

Parallel dazu wird eine wäßrige Kollagensuspension mit einer Viskosität von 8 Pa x s bei 25 °C aus 2 Gew.-% unlöslichem Kollagen Typ 1, isoliert aus Rinderhäuten, und 2 Gew.-% Ascorbinsäure hergestellt. Der pH-Wert der Suspension wird mit Salzsäure auf 3,4 eingestellt. Diese Suspension wird auf eine rechteckige Folie 47 mit den Abmessungen 350 mm x 19 mm in einer gleichmäßigen Schichtstärke von 1 mm mit einer Rakel aufgetragen.

Die Folie 47 besitzt eine ausreichende Festigkeit und besteht beispielsweise aus Teflon. Vorteilhaft ist, wenn die kürzeren Seiten der Folie 47 mit jeweils einer Vertiefung 48, 49 versehen sind, deren Abstand dem des Ringes 43 und des Flanschs 44, zwischen welchen die schlauchförmige Fremdstruktur 40 gespannt liegt, entspricht. Durch die Tiefe der Vertiefungen 48, 49 und durch eine Änderung des Flanschs 44 und des Rings 43 kann außerdem die Wandstärke der Probe festgelegt werden.

Anschließend werden der Ring 43 und der Flansch 44 der Metallrohre 41, 42 in die Vertiefungen 48, 49 der mit der Kollagensuspension beschichteten Folie 47 gesetzt und die Folie 47 auf einem ebenen Tisch um die Anordnung aus schlauchförmiger Fremdstruktur 40 und Metallrohranschlüssen gewickelt. Dadurch wird eine Form für die zu fertigende Gefäßprothese gebildet, mit dem Flansch 44 und dem Ring 43 als Seitenbegrenzung und der Folie 47 als Außenbegrenzung und gleichzeitige Isolationsschicht. Daraufhin werden die Metallrohre über ihre freien Enden an eine Vakuumpumpe angeschlossen. Mit Hilfe der Vakuumpumpe wird ein leichter Unterdruck von 100 mbar angelegt und die Suspension in die Poren des ePTFE-Schlauchs gesaugt. Anschließend wird das eine Metallrohr 41, das einen Außendurchmesser von 4 mm hat, durch den Ring 43 bis zum Ende des anderen Metallrohres 46 vorgeschoben. Am Ende des Metallrohres 41 befindet sich eine Dichtung 55, so daß an dieser Verbindungsstelle (Metallrohr 41 zu Metallrohr 42) keine Kühlflüssigkeit austreten kann.

Anschließend wird die Kollagensuspension durch einen einseitig kontrollierten Einfriervorgang gerichtet erstarrt. Dazu wird die Temperatur des Metallrohres 41, welches nun als Kühlrohr fungiert, von Raumtemperatur (ca. 25 °C) auf -50 °C mit einer konstanten Kühlrate von 6 K/min abgesenkt. Als Kühlmedium dient Isopropanol, zur gleichmäßigeren Temperaturverteilung entlang dem Metallrohr wird das Kühlmedium geteilt und im Gegenstrom durch die zwei konzentrisch angeordneten Lumina zum Thermostat zurückgeführt.

Nach dem Einfrieren wird die Folie 47 entfernt. Die Proben werden bei -70 °C mindestens 12 h gelagert und anschließend gefriergetrocknet. Dabei wird die Kondensatortemperatur auf -85 °C gehalten, bis der Wassergehalt der entstandenen Kollagenmatrix < 10 Gew.-% ist. Im Anschluß wird die Kollagenmatrix der Form entnommen und bei 5 x 10⁻⁵ bar Unterdruck für ca. 14 h einer Temperatur von 106 °C ausgesetzt, um das Kollagen dehydrotherm zu vernetzen.

Die Enden der Proben werden dann jeweils im Abstand von 20 mm vom Probenrand abgetrennt.

Nach einer darauffolgenden Sterilisation ist die nach dem erfindungsgemäßen Verfahren hergestellte Gefäßprothese aus Kollagen mit ePTFE-Verstärkung zur Besiedelung mit Myofibroblasten und Endothelzellen in einem Zellreaktor bereit.

## Patentansprüche

1. Verfahren zur Herstellung eines Implantats mit einer strukturierten Fremdstruktur und einer in der strukturierten Fremdstruktur zumindest teilweise verankerten porösen Proteinmatrix mit gerichteter Porenstruktur, wobei als Fremdstruktur ein Material verwendet wird, das ausgewählt ist aus einem strukturierten beständigen künstlichen Material, einem strukturierten resorbierbaren künstlichen Material und einem strukturierten natürlichen Material oder Mischungen der genannten Materialien, mit den Schritten:
a) Bereitstellen der Fremdstruktur,
b) Auftragen einer Suspension, Dispersion oder Paste mit Kollagen und löslichen, nicht-kollagenen Bestandteilen,
c) zumindest teilweises Einbringen der Suspension, Dispersion oder Paste in die Fremdstruktur durch Druck, Vakuum oder Zentrifugation,
d) einseitiges Abkühlen einer Oberfläche der Suspension, Dispersion oder Paste und gleichzeitiges Isolieren der anderen Oberfläche.

2. verfahren nach Anspruch 1, **dadurch gekennzeichnet, daß** als strukturierte Fremdstruktur ein beständiges künstliches Material verwendet wird, das aus einer Gruppe, umfassend Polytetrafluorethylen, Polyurethan, Polystyrol oder Polyester, Keramik oder Metall ausgewählt ist.

3. verfahren nach Anspruch 1, **dadurch gekennzeichnet, daß** als strukturierte Fremdstruktur ein resorbierbares künstliches Material verwendet wird, das aus einer Gruppe, umfassend Poly-Lactid, Poly-Glykolsäure, Polyhydroxyalkanoate oder deren Copolymere, ausgewählt ist.

4. Verfahren nach Anspruch 1, **dadurch gekennzeichnet, daß** als strukturierte Fremdstruktur ein natürliches Material verwendet wird, das aus einer Gruppe, umfassend Polysaccharide wie bspw. Chitin, Cellulose, Kollagen oder Hydroxylapatite wie bspw. Calciumphosphat, ausgewählt ist.

5. Verfahren nach einem der Ansprüche 1 bis 4, **dadurch gekennzeichnet, daß** die Porengröße der Proteinmatrix zwischen 5 µm und 500 µm ausgebildet wird.

6. Verfahren nach einem der Ansprüche 1 bis 5, **dadurch gekennzeichnet, daß** die Proteinmatrix nach dem Abkühlen gefriergetrocknet wird.

7. Verfahren nach einem der Ansprüche 1 bis 6, **dadurch gekennzeichnet, daß** die Schichtdicke der Proteinmatrix zwischen 0,1 und 5 cm ausgebildet wird.

8. Verfahren nach einem der Ansprüche 1 bis 7, **dadurch gekennzeichnet, daß** das Implantat nach der Gefriertrocknung sterilisiert wird.

9. Verfahren nach einem der Ansprüche 1 bis 8, **dadurch gekennzeichnet, daß** das Implantat vor der Implantation mit Zellen besiedelt wird.

10. Verfahren nach einem der Ansprüche 1 bis 9, **dadurch gekennzeichnet, daß** das Implantat vor der Implantation mit einem Wirkstoff beladen oder beschichtet wird, der aus der Gruppe, umfassend Hirudin, Aspirin, Heparansulfat oder Albumin, ausgewählt ist.

11. Implantat mit einer strukturierten Fremdstruktur und einer darin zumindest teilweise verankerten porösen Proteinmatrix mit gerichteter Porenstruktur, wobei als Fremdstruktur ein Material verwendet wird, das ausgewählt ist aus einem strukturierten beständigen künstlichen Material, einem strukturierten resorbierbaren künstlichen Material und einem strukturierten natürlichen Material oder Mischungen der genannten Materialien.

12. Implantat nach Anspruch 11, **dadurch gekennzeichnet, daß** es mit dem Verfahren nach einem der Ansprüche 1 bis 10 hergestellt wird.

## Claims

1. Method for producing an implant having a textured foreign structure and a porous protein matrix which is at least partially anchored therein and which has an orientated pore structure, wherein as foreign structure a material is employed which is selected from a textured stable synthetic material, a textured absorbable synthetic material and a textured natural material or mixtures of said materials, comprising the steps of:
a) providing of said foreign structure
b) applying a suspension, dispersion or paste containing collagen and soluble, non-collagenous constituents,
c) introducing, at least partially, said suspension, dispersion or paste into said foreign structure by means of pressure, vacuum or centrifugation,
d) unilaterally cooling a surface of said suspension, dispersion or paste and at the same time insulating the other surface.

2. Method according to Claim 1, **characterized in that** said textured foreign structure employed is a stable synthetic material which is selected from a group comprising polytetrafluoroethylene, polyurethane, polystyrene or polyester, ceramic or metal.

3. Method according to Claim 1, **characterized in that** said textured foreign structure employed is an absorbable synthetic material which is selected from a group comprising polylactide, polyglycolic acid, polyhydroxyalkanoate or their copolymers.

4. Method according to Claim 1, **characterized in that** said textured foreign structure employed is a natural material which is selected from a group comprising polysaccharides, such as chitin or cellulose, collagen or hydroxylapatites, such as calcium phosphate.

5. Method according to any one of Claims 1 to 4, **characterized in that** the size of the pores of the protein matrix which are formed is between 5 µm and 500 µm.

6. Method according to any one of Claims 1 to 5, **characterized in that** the protein matrix is freeze-dried after having been cooled.

7. Method according to any one of Claims 1 to 6, **characterized in that** the thickness of the protein matrix layer which is formed is between 0.1 and 5 cm.

8. Method according to any one of Claims 1 to 7, **characterized in that** the implant is sterilized after having been freeze-dried.

9. Method according to any one of Claims 1 to 8, **characterized in that** the implant is colonized with cells prior to implantation.

10. Method according to any one of Claims 1 to 9, **characterized in that**, prior to implantation, the implant is loaded or coated with an active compound which is selected from the group comprising hirudin, aspirin, heparan sulphate and albumin.

11. Implant having a textured foreign structure and a porous protein matrix which is at least partially anchored therein and which possesses an orientated pore structure, wherein as foreign structure a material is employed which is selected from a textured stable synthetic material, a textured absorbable synthetic material and a textured natural material or mixtures of said materials.

12. Implant according to Claim 11, **characterized in that** it is produced using the process according to any one of Claims 1 to 10.

## Revendications

1. Procédé d'obtention d'un implant comportant une structure étrangère structurée et une matrice protéique poreuse, ancrée au moins partiellement dans la structure étrangère structurée et comportant une structure de pores orientée, selon lequel pour la structure étrangère on utilise une matière qui est choisie parmi une matière synthétique stable structurée, une matière synthétique résorbable structurée et une matière naturelle structurée ou des mélanges des matières citées, lequel procédé comporte les étapes :
a) mise à disposition de la structure étrangère,
b) application d'une suspension, dispersion ou pâte avec un collagène et des constituants solubles non collagéniques,
c) introduction au moins partielle de la suspension, dispersion ou pâte dans la structure étrangère par pression, sous vide ou par centrifugation,
d) refroidissement unilatéral d'une surface de la suspension, dispersion ou pâte et isolation simultanée de l'autre surface.

2. Procédé selon la revendication 1, **caractérisé en ce que** pour la structure étrangère structurée on utilise une matière synthétique stable, qui est choisie dans le groupe constitué par le polytétrafluoréthylène, le polyuréthanne, le polystyrène ou le polyester, une céramique ou un métal.

3. Procédé selon la revendication 1, **caractérisé en ce que** pour la structure étrangère structurée on utilise une matière synthétique résorbable, qui est choisie dans un groupe contenant des polylactides, des acides polyglycoliques, des polyhydroxyalcanoates ou des copolymères de ceux-ci.

4. Procédé selon la revendication 1, **caractérisé en ce que** pour la structure étrangère structurée on utilise une matière naturelle, qui est choisie dans le groupe constitué par les polysaccharides, tels que la chitine, la cellulose, le collagène, ou des hydroxylapatites, tels que le phosphate de calcium.

5. Procédé selon l'une quelconque des revendications 1 à 4, **caractérisé en ce que** la grosseur des pores de la matrice protéique se situe entre 5 µm et 500 µm.

6. Procédé selon l'une quelconque des revendications 1 à 5, **caractérisé en ce que** la matrice protéique après le refroidissement est lyophilisée.

7. Procédé selon l'une quelconque des revendications 1 à 6, **caractérisé en ce que** l'épaisseur de couche de la matrice protéique se situe entre 0,1 et 5 cm.

8. Procédé selon l'une quelconque des revendications 1 à 7, **caractérisé en ce que** l'implant est stérilisé après la lyophilisation.

9. Procédé selon l'une quelconque des revendications 1 à 8, **caractérisé en ce que** l'implant a été colonisé avec des cellules avant sa mise en place.

10. Procédé selon l'une quelconque des revendications 1 à 9, **caractérisé en ce que** l'implant avant sa mise en place a été chargé ou revêtu d'une substance active, qui est choisie dans le groupe contenant l'hirudine, l'aspirine, l'héparansulfate ou l'albumine.

11. Implant comportant une structure étrangère structurée et une matrice protéique poreuse, ancrée au moins partiellement dans cette dernière et comportant une structure de pores orientée, selon lequel pour la structure étrangère on utilise une matière qui est choisie parmi une matière synthétique stable structurée, une matière synthétique résorbable structurée et une matière naturelle structurée ou des mélanges des matières citées.

12. Implant selon la revendication 11, **caractérisé en ce qu'**il est réalisé par le procédé selon l'une quelconque des revendications 1 à 10.
